# EUROPEAN PATENT SPECIFICATION

(11) **EP 1 743 651 B1**
(45) Date of publication and mention of the grant of the patent: **07.01.2009**
(21) Application number: 05729588.3
(22) Date of filing: 18.03.2005
(51) Int. Cl.: A61K 39/00, A61K 39/04, A61P 37/00, A61P 35/00, A61P 15/00

(54) **VACCINE COMPOSITIONS WHICH ARE OBTAINED FROM STREPTOMYCES**
VAKZINE-ZUSAMMENSETZUNGEN AUS STREPTOMYCES
COMPOSITIONS VACCINALES OBTENUES A PARTIR DE STREPTOMYCES

(30) Priority: 18.03.2004 CU 4005104
(43) Date of publication of application: 17.01.2007
(73) Proprietor: Instituto Finlay, Centro de Investigacion-Produccion de vacunas y sueros, La Coronela, La Lisa, Ciudad Habana 11600 (CU); Centro de Quimica Farmaceutica, 11200 Ciudad de La Habana (CU)
(72) Inventor: SARMIENTO GARCIA SAN MIGUEL, Maria Elena, 11300 Ciudad de La Habana (CU); ACOSTA DOMINGUEZ, Armando, 11300 Ciudad de La Habana (CU); VALLIN PLOUS, Carlos, Román, 11200 Ciudad de La Habana (CU); OLIVARES ARZUAGA, Nesty, 10700 Ciudad de La Habana (CU); LOPEZ HERNANDEZ, Yamilé, 11000 Ciudad de La Habana (CU); RODRIGUEZ VALDES, Caridad, 12000 Ciudad de la Habana (CU); MARTINEZ BENITEZ, Máximo, 11200 Ciudad de La Habana (CU); GONZALEZ MESA, Leonora, 11500 Ciudad de La Habana (CU); INFANTE BOURZAC, Juan, Francisco, 11200 Ciudad de La Habana (CU); RAMOS MORI, Astrid, 11900 Ciudad de La Habana (CU)
(74) Representative: Gil-Vega, Victor
(86) International application number: PCT/CU2005/000002
(87) International publication number: WO 2005/087259

(56) References cited:
- EP-A- 0 148 552
- EP-A- 0 962 532
- DATABASE WPI Section Ch, Week 200379 Derwent Publications Ltd., London, GB; Class B04, AN 2003-849754 XP002334575 KIM C J ET AL.: "Microorganism Streptomyces sp. amlk-335 producing cyclo(proline-phenylalanine) and cyclo(leucine-proline) useful for preparing an antimicrobial and anticancer composition" & KR 2003 055 089 A (KOREA RES INST BIOSCIENCE & BIOTECHNOLOG) 2 July 2003 (2003-07-02)
- DONALD TREMBLAY ET AL.: "High-level hetrologous expression and secretion in Streptomyces lividans of two major antigenic proteins from Mycobacterium tuberculosis" CANADIAN JOURNAL OF MICROBIOLOGY, vol. 48, 2002, pages 43-48, XP002334573

## Description

### Field of the Invention

The present invention relates to the field of immunology, specifically to the control of infectious diseases caused by mycobacteria using vaccines developed from live strains of Streptomyces, expressing or not antigens of *M. tuberculosis,* which demonstrated their protective capacity against challenge with BCG and *M*. *tuberculosis* after being administered by different routes.

### Background of the invention

Among mycobacteria, important pathogens for animals and men are found: *Mycobacterium tuberculosis* which causes tuberculosis, *Mycobacterium leprae* responsible for leprosy, *Mycobacterium avium* and *Mycobacterium intracelulare* which cause tuberculosis in immunodepressed patients as well as other mycobacteria which cause diseases in humans, although to a lesser degree (Somner HM, Good RC. Mycobacterium. In: Manual of clinical Microbiology, 4 ed. Washington D.C: A Society for Microbiology; 1985. p.216-248., Orme IM. Immunity to mycobacteria. Current Opinion in Immunology. 1993; 5: 497-502).

In the case of animals, *Mycobacterium avium* subsp. pararatuberculosis causing Jones Disease in ruminants and *Mycobacterium bovis* causing tuberculosis in cattle highlight. (Dannenberg Am. Pathogenesis of tuberculosis: native and acquired resistance in animals and humans. In Leive L, Schelesinger D (eds). Microbiology. 1984, p344-354).

Tuberculosis (TB) is among the most important mycobacterial diseases in men. It constitutes a world health problem and it is the leading cause of death associated to infectious diseases despite vaccination with BCG and the use of a great number of drugs for its control (Dolin PJ, Raviglione MK, Kochi A. Global tuberculosis incidence and mortality during 1990-2000. Bull WHO. 2001; 72: 213).

It is estimated that the third part of the world population has been infected by *Mycobacterium tuberculosis.* All over the world, 8 million people develop active TB every year and three million dies. Co-infection with the Human Immnunodeficiency Virus (HIV) represents 3 to 5 % of the cases (Dolin PJ, Raviglione MK, Kochi A. Global tuberculosis incidence and mortality during 1990-2000. Bull WHO. 1994; 72: 213).

Due to the great spread of the disease, new and better diagnosis methods, vaccine preparations and therapeutical agents are required (Collins FM. Tuberculosis: The Return of an Old Enemy. Critical Reviews in Microbiology. 1993; 19: 1-16).

Treatment is based on drugs combinations administered in relatively high doses for long periods of time with associated toxicity. This makes difficult the implementation of programs of controlled treatment (McCarthy M. Experts see progress in fight against tuberculosis Lancet. 2002; 359:2005). In this regard, the decrease of treatment times favoring the application of control programs and their fulfillment that would avoid the appearance of resistant strains, is desirable. Decreasing doses of pharmaceuticals used would also be a useful element to diminish the treatment toxicity.

Currently, the appearance of strains with multiple resistance to drugs is an increasing problem which claims the development of new therapeutical alternatives for the high number of infected individuals (50 millions) and for the increasing number of patients with these characteristics occurring in the future (McCarthy M, News. Experts see progress in fight against tuberculosis. Lancet. 2002; 359:2005; Hopewell PC. Tuberculosis Control: How the world has changed since 1990. Bull. World Health Org 2002, 80:427, Freire M, Rosigno G Joining forces to develop weapons against TB: together we must. Bull. World Health Org 2002, 80:429).

Additionally, there exist multiple species of mycobacteria causing diseases in man for which an adequate treatment is not available.

BCG is the only tuberculosis vaccine currently available for human use. Almost three billion doses have been applied all over the world. Its efficacy widely varies depending on the strain used, nutritional status, genetic background, aging and presence of intercurrent infections. Its use is considered only effective to prevent the serious forms of the disease (miliary and meningitis) in infancy but not to prevent pulmonary tuberculosis; so it is urgent to develop new vaccine preparations (Hirsch LS, Johnson-JL, Ellner JJ. Pulmonary tuberculosis. Curr-Opin-Pulm-Med1999;5(3):143-50; Jacobs GG, Johonson JL, Wallis RS. Tuberculosis vaccines: how close to human testing. Tuber Lung Did 1997;78:159-169; Ginsberg AM. What's new in tuberculosis vaccines? Bull. World Health Org 2002, 80:483).

The most important strategies to develop vaccines against tuberculosis include the use of inactivated strains, genetically or not attenuated strains, nucleic acids vaccines, subunits vaccines and attenuated live strains expressing antigens of *M. tuberculosis.*

Since inactivated vaccines are composed of dead microorganisms, they present the disadvantage of having a decreased protective capacity due to the impossibility of persistence in vivo and to produce relevant proteins for protection as the secreted ones.

The attenuated strains present as disadvantage the possibility of reversion to virulence after being administered which is of concern with regard to safety of humans.

Nucleic acids vaccines, despite being a promising strategy so far, have not achieved adequate immunogenicity levels in humans.

Sub-units vaccines are considered not to have the same immunogenicity potential as live microorganisms because they are purified components from the microorganism or obtained by recombinant methods which makes difficult to achieve protective responses, mainly cell responses of T-cell type, T-helper type 1 (TH 1).

The strategy of antigen expression of vaccine interest in attenuated live strains is one of the most promising strategies in the field of new generation vaccines against tuberculosis. An important element of this strategy is the selection of the expression vector, which depending on the selected strain, could have complications from the regulatory point of view, similar to those faced with the use of attenuated live strains.

Relevant background art are also DATABASE WPI Section Ch, Week 200379 Derwent Publications Ltd., London, GB; Class B04, AN 2003-849754 XP002334575 KIM C J ET AL.: "Microorganism Streptomyces sp. amlk-335 producing cyclo(proline-phenylalanine) and cyclo(leucine-proline) useful for preparing an antimicrobial and anticancer composition" & KR 2003 055 089 A (KOREA RES INST BIOSCIENCE & BIOTECHNOLOG) 2 July 2003 (2003-07-02) where it is disclosed the therapeutic use as antimicrobial and anticancer composition of compounds isolated from Streptomyces and DONALD TREMBLAY ET AL.: "High-level hetrologous expression and secretion in Streptomyces lividans of two major antigenic proteins from Mycobacterium tuberculosis" CANADIAN JOURNAL OF MICROBIOLOGY, vol. 48, 2002, pages 43-48, XP002334573, that discloses the heterologous production and secretion of two mycobacterial antigens by Streptomyces lividans.

### Description of the invention

Taking into account that *Streptomyces* and *M. tuberculosis* belong to the same class and share a great quantity of genes and antigens together with the proven innocuity of *Streptomyces* for men, the wide use of these bacteria to produce pharmaceuticals for human use as well as the big development of methods for the expression of heterologous proteins in this system, including proteins of M. tuberculosis, the development of vaccines against tuberculosis using as active principles live strains of *Streptomyces* that can express or not antigens of *M. tuberculosis* administered by different routes, including the mucosal, was designed by the present invention.

The vaccine preparations of the present invention comprise a variety of active principles derived from the microorganism *Streptomyces.* Among them, we have:
- *Streptomyces* (wild strain)
- Recombinant *Streptomyces* expressing the antigen Apa of *M. tuberculosis*

The wild strain used in the present invention is a non-pathogenic industrial strain, widely used in the production of medicines for man.

A marked humoral and cellular immunogenicity of strains after their administration by different routes was surprisingly observed. The responses obtained were directed against the antigens of the strain used in the immunization (*Streptomyces*)*,* against the antigen of *M. tuberculosis* expressed (Apa) and against other antigens of *M. tuberculosis* and BCG (Example 1), which confirmed the antigen community existing between *Streptomyces* and Mycobacteria and their wide cross-reactivity. This fact guaranteed the use of these strains as vaccines against *M. tuberculosis.*

Another fact guaranteeing their use is their incapability to colonize and cause histopathological lesions in hosts, which reaffirms their innocuity (Example 2)

These strains are prophylactically applicable to prevent tuberculosis. Induction of a protective condition against *M. tuberculosis* and BCG was shown in all the administration routes used (Example 3).

The compositions of the present invention produced a significant decrease in the levels of pulmonary infection with BCG and *M. tuberculosis* in an infection model in mice (Example 3).

The present invention approaches in a novel way the prevention of diseases caused by mycobacteria, in particular against tuberculosis, using vaccines based on Streptomyces strains. It is particularly novel the use of Streptomyces strains, not known at all in the state-of-the-art. It is also novel the fact that these strains were effective both by mucosal and parenteral route.

It was significant the fact that the Streptomyces strains can be used as live vectors of antigen expression of vaccine interest that has not been reported in the state-of the art., this broadening the possibility to use these strains for the expression on non-mycobacterial antigens allowing their use to prevent and treat allergic, tumoral and autoimmune diseases and to prevent pregnancy.

### Brief description of the drawings

**Figure 1****:** Western blot. Nitrocellulose strips with extracts of *S. lividans* (1) and BCG(2) were studied against a pool of sera of animals immunized with *S. lividans* (Group 2)
**Figure 2****:** Western blot. Nitrocellulose strips with extracts of BCG (1) and S. *lividans* (2) were studied against a pool of sera of animals immunized with BCG (Group 3)
**Figure 3****:** Western blot. Nitrocellulose strips with extracts of BCG (1) and *S. lividans* (2) were studied against a pool of sera of animals immunized with Saline Solution (Group 1)
**Figure 4**: Challenge experiment with BCG. The values represent the mean log of the CFU/mg of lung tissue. There were statistical differences (p<0.05) between the group immunized with Streptomyces *(S. lividans)* and the control group immunized with Saline Solution (CN).

### Examples

The present invention will be described through the following specific examples:

### Example 1. Immunogenicity study

### Animals:

Male, 8-10 weeks old Balb/c mice supplied by CENPALAB, Cuba, were used in the experiments.

### BCG

Lyophilized, live attenuated BCG. InterVax, Biological Limited, Canada.

### Streptomyces lividans

Strain 1326, untransformed *(Streptomyces)* and transformed genetically expressing the ApA protein of *Mycobacterium tuberculosis (Streptomyces-ApA)* were used in the experiments.

### Immunization schedulle

40 Balb/c mice were divided into 4 groups of 10 animals each. (Table 1).
Group 1 received SS and was used as control.
Animals from groups 2 and 3 received 10⁵ CFU of *Streptomyces* via IP at 3 week intervals. The animals of the group 3 received *S*. *lividans* expressing the ApA protein of *M. tuberculosis.*

The animals of group 4 were immunized with the same schedule but using 10⁵ of BCG in each immunization; 21 days after the last immunization blood samples were taken fro each animal.

**Table 1: Immunogenicity study**

| **GROUP** | **ROUTE** | **INOCULUM** | **N** |
|---|---|---|---|
| | IP | SSF | |
| 1 | | 200 µL | 10 |
| 2 | | *Streptomyces* | |
| | | 10⁵ CFU | 10 |
| 3 | | Streptomyces-ApA | |
| | | 10⁵ CFU | 10 |
| | | BCG | |
| 4 | | 10⁵ CFU | 10 |

### Western Blot

Protein extracts of *S. lividans* and BCG and recombinant Apa protein of *M. tuberculosis* were separated by SDS-PAGE (Laemmli A, UK. Nature 1970; 227(6): 680-685) and blotted to a 0.45 mcm nitrocellulose membrane using a semi-dry system (NovaBlot II, Pharmacia, Sweeden).

The membranes were blocked with BSA, 2% in PBS for 2 hours at 37, washed and incubated for 1 hour at 37 with polls of sera of the animals of groups 2, 3 and 4 diluted 1.150 in PBS.
After the wash, the membranes were incubated with a polyvalent anti mouse peroxidase conjugate (Sigma), diluted 1.1500 for 1 hour at 37 and developed with Diaminobenzidine and H₂O₂ as substrate.

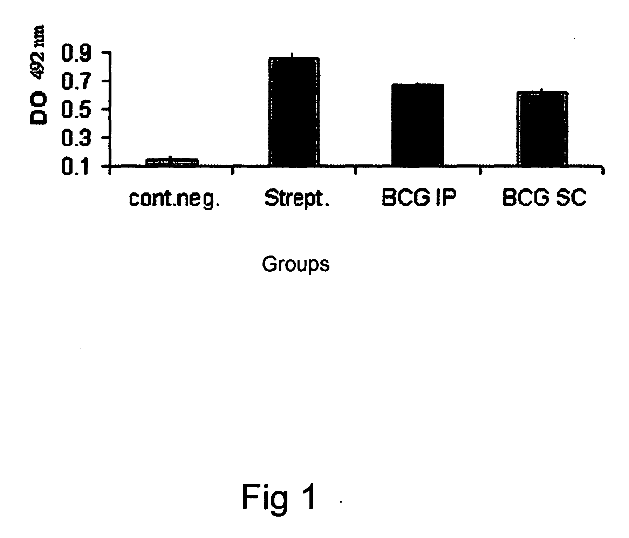

The result obtained from the immunogenicity study demonstrated the induction of specific antibody responses against the antigens of *S*. *lividans* in the animals immunized with the microorganism (**Figure 1**).

Additionally, the immunized animals recognized proteins of BCG, demonstrating the cross reactivity with mycobacteria of the immune response elicited (**Figure 1**).

This result s highly relevant, demonstrating the immunizing potential of Streptomyces against mycobacteria.

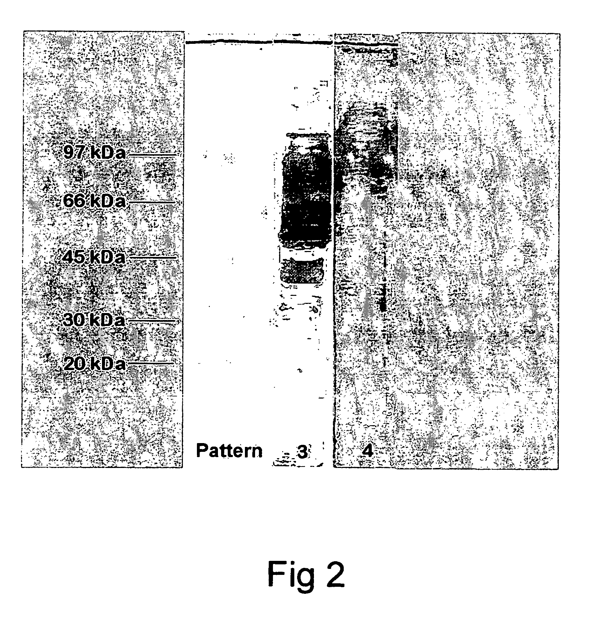

The cross reactivity against Streptomyces of the response elicited against BCG was too demonstrated (**Figure 2**).

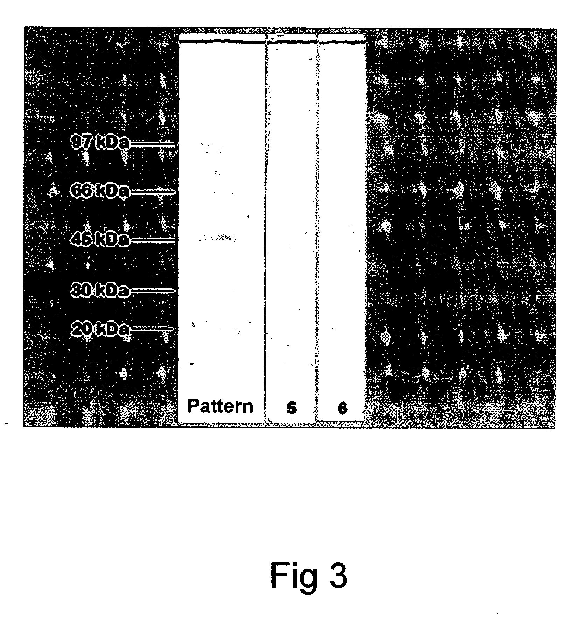

The animals sera immunized with Saline Solution were not reactive against antigens of Streptomyces or BCG (**Figure 3**).

The group of animals immunized with Streptomyces expressing the Apa protein of M. tuberculosis showed a similar response than the animals immunized with the non transformed Streptomyces (data non shown). In this group of animals an specific immune response was demonstrated against the Apa protein by Western Blot (data non shown).

This result demonstrated the capability of Streptomyces to be used as live vector for the expression of heterologous antigens, in particular from *M. tuberculosis.*

### Example 2. Biodistribution study

### Animals:

Male, 8-10 weeks old Balb/c mice supplied by CENPALAB, Cuba, were used in the experiments.

### BCG

Lyophilized, live attenuated BCG. InterVax, Biological Limited, Canada.

### Streptomyces lividans

Strain 1326, untransformed and transformed genetically expressing the ApA protein of *Mycobacterium tuberculosis* were used in the experiments.
The study was designed with 48 mice, distributed in 8 groups of 6 animals (Table 2) Animals from group 1 received 200µl of Saline Solution (SS) IP and the animals of group 5 received 50 µl of Saline Solution IN.
Animals form the groups 2, 3 and 4 received *S. lividans* in doses of 10⁵, 10³ and 10² respectively in 200 µl of distilled water by the intraperitoneal route (IP).

The animals form the groups 6, 7 and 8 received *S. lividans* in doses of 10³, 10² and 10¹ in 50 µl of distilled water by the intranasal route (IN).
After 30 days the animals were sacrificed and the heart, lung, liver. Spleen and kidney were studies microbiologically (3 animals) and histopathologically (3 animals).
The histopathological study was made with tissue samples stained with Haematoxilin and Eosin.
The microbiological studies were carried out with YEME medium (Tobias Kieser, Mervyn J. Viv., Mark J. Buttner, Perth F. Charter, David A. Hopwood. Practical Sytreptomyces Genetics. Crowes, Norwich. England. 2000).

**Tabla 2: Biodistribution of Streptomyces lividans in Balb/c mice**

| **GROUP** | **ROUTE** | **DOSE** | **ORGAN** |
|---|---|---|---|
| 1 | IP | SSF 200 µL | Heart Lung Liver Spleen Kidney |
| 2 | | 10⁵ CFU | |
| 3 | | 10³ CFU | |
| 4 | | 10² CFU | |
| 5 (negative control) | IN | SSF 50 µL | |
| 6 | | 10³ CFU | |
| 7 | | 10² CFU | |
| 8 | | 10¹ CFU | |

In the biodistribution study the presence of the microorganism was not evident in the organs studied

The histopathological study did not demonstrate lesions in the organs studied. Similar results were obtained in the study of Streptomyces expressing the Apa protein of M. tuberculosis (data non shown).

Taking into consideration these results, we can conclude that Streptomyces is safe, demonstrating the feasibility of their use as live vaccine without adverse effects.
It is important to highlight the fact that despite the safety of the tested strains, they elicited a good immune response (**Figure 1**).

### Example 3. Challenge experiments

### Animals:

Male, 8-10 weeks old Balb/c mice supplied by CENPALAB, Cuba, were used in the experiments.

### BCG

Lyophilized, live attenuated BCG,. InterVax, Biological Limited, Canada.

### Streptomyces lividans

Strain 1326, untransformed and transformed genetically expressing the ApA protein of *Mycobacterium tuberculosis* were used in the experiments.
Where studied 26 animals distributed in 3 groups. (Table 3).
The animals were immunized IP 3 times at 2 week intervals.
Group 1 with SS, Group 2 with 10⁵ CFU of *S*. *lividans* and Group 3 with 10⁵ CFU of BCG. 3 weeks after the last immunization the animals were challenged with 0.5 x 10⁶ CFU of BCG by the IN route. 24 hours later, the animals were sacrificed and the lungs obtained for microbiological studies.

### Microbiological studies.

Lung macerates where plated in Ogawa medium (Manual de la OXID. Cuarta Edición. 1981 Editado por OXID Limited, England) and incubated for 28 days at 37. After the incubation period, the CFU were counted and the CFU number/ mg of lung tissue were determined.

### Statistical processing

The statistical comparison between groups was made with the Kruskal-Wallis test and the Test of multiple comparisons of free distribution was used as complementary test.

**Table 3: Challenge experiment**

| **GROUP** | **INOCULUM AT 0, 0, 21 and 42 DAYS** | **CHALLENGE DAY 63** | **N** |
|---|---|---|---|
| 1 | SSF 200 µL | BCG 0,5 x 10⁶ CFU | 8 |
| 2 | *Streptomyces lividans* 10⁵ CFU | | 10 |
| 3 | BCG 10⁵ CFU | | 8 |

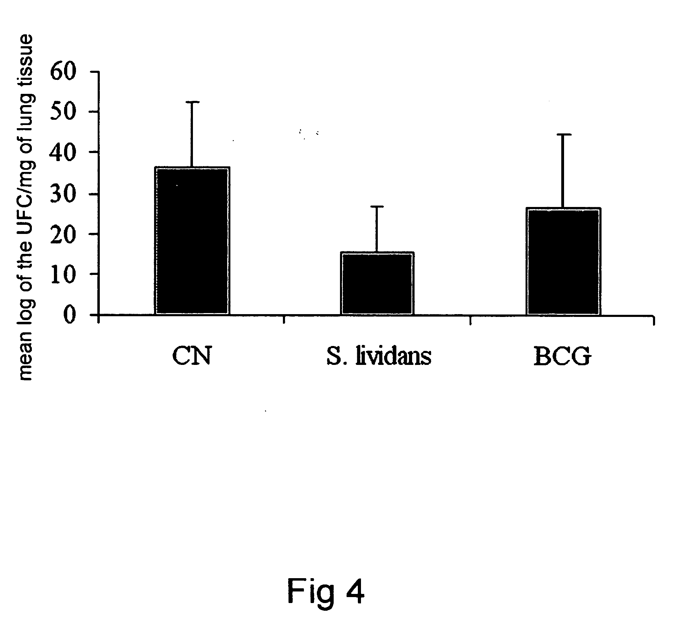

In the group immunized with *Streptomyces* there was a statistical decrease in the CFU of BCG in lungs compared with the animals immunized with BCG and the control group (Figure 4). Similar results were obtained in the group immunized with *Streptomyces* expressing the Apa protein of *M. tuberculosis* (data non shown).

Groups of animals immunized with transformed and non transformed *Streptomyces* were protected upon challenge with *M. tuberculosis* (data non shown).

The above results demonstrated the protective capacity of Streptomyces against mycobacteria and support their use as vaccines for the prevention of mycobacterial infections.

### Advantages of the proposed solution.

The advantage of using this kind of strains as vaccines against mycobacterial infections, specially against tuberculosis, is the use of non-pathogenic strains allowing the use of live strains in humans, which guarantees an adequate immunogenicity and stimulation of immune responses for protection.

The wide cross protective capacity, demonstrated against BCG and *M. tuberculosis* is an important advantage for their use as vaccine against several mycobacterial infections.
The wide knowledge of the genetics of Streptomyces and the availability of genetic methods for their transformation and expression of high levels of heterologous antigens are additional advantages for their use as recombinant live attenuated vectors. The mucosal administration route ensures an easy and versatile application way at the entrance site of mycobacteria favoring the blocking of infection and therefore the prophylactic effect.
Another advantage lies in the wide experience in the industrial use of these strains to produce medicines for human use, guaranteeing the industrial production of these vaccines.
The genetically transformed Streptomyces strains, expressing antigens of non-mycobacterial vaccine interest, can be used for the prophylaxis or therapeutics of non-mycobacterial infectious, autoimmune, allergic and tumoral diseases and prevention of pregnancy.

## Claims

1. Vaccine composition obtained from Streptomyces **characterized by** having as active principle one or more wild strains of Streptomyces genus or mutant or recombinant strains derived from such strains, as well as an adequate excipient.

2. Vaccine composition according to claim 1 wherein such Streptomyces strains are live strains.

3. Vaccine composition according to claims 1 and 2 wherein such strains of Streptomyces are *Streptomyces lividans,* Streptomyces coelicolor and *Streptomyces Sp*.

4. Vaccine composition according to claim 1 wherein such recombinant strains of Streptomyces express one or more heterologous antigens.

5. Vaccine composition according to claim 4 **characterized** wherein such recombinant strains of Streptomyces express one or more heterologous antigens of *Mycobacterium Tuberculosis.*

6. Vaccine composition of claims 1 to 5 for the prevention or therapeutics of infectious diseases.

7. Vaccine composition of claim 6 for the prevention or therapeutics caused by mycobacteria.

8. Vaccine composition of claim 7 for the prevention or therapeutics of tuberculosis.

9. Vaccine composition of claims 1 to 5 for the prevention or therapeutics of tumoral diseases.

10. Vaccine composition of claims 1 to 5 for the prevention or therapeutics of auto-immune diseases.

11. Vaccine composition of claims 1 to 5 for the prevention or therapeutics of allergic diseases.

12. Vaccine composition of claims 1 to 5 for the prevention of pregnancy.

## Patentansprüche

1. Imstoffzusammensetzung ausgehend vom Streptomyces, **dadurch gekennzeichnet dass** sie als Wirkstoff einen oder mehrer Wildstämme von Streptomyces genus oder mutant oder rekombinant Stämmen enthalten, die von diesen Stämmen abgeleitet sind, sowie einen passenden Träger.

2. Imstoffzusammensetzung nach Anspruch 1, **dadurch gekennzeichnet, dass** die Streptomyces-Stämme lebende Stämme sind.

3. Imstoffzusammensetzung nach Anspruch 1 und 2, **dadurch gekennzeichnet dass** diese Streptomyces-Stämme *Streptomyces lividans* sind, Streptomyces coelicolor und *Streptomyces Sp.*

4. Impfstoffzusammensetzung nach Anspruch 1, **dadurch gekennzeichnet dass** diese rekombinanten Streptomyces-Stämme ein oder mehrere heterologe Antigene ausdrücken.

5. Impfstoffzusammensetzung nach Anspruch 4, **dadurch gekennzeichnet dass** diese rekombinanten Streptomyces-Stämme ein oder mehrere heterologe Antigene von *Mycobacterium Tuberculosis* ausdrücken.

6. Impfstoffzusammensetzung nach Anspruch 1 bis 5 für die Vorbeugung oder terapeutische Behandlung von Infektionskrankheiten.

7. Impfstoffzusammensetzung nach Anspruch 6 für die Vorbeugung oder terapeutische Behandlung von mykobakterielle Erkrankungen.

8. Impfstoffzusammensetzung nach Anspruch 7 für die Vorbeugung oder terapeutische Behandlung der Tuberkulose.

9. Impfstoffrzusammensetzung nach Anspruch 1 bis 5 für die Vorbeugung oder terapeutische Behandlung von Tumorerkrankungen.

10. Impfstoffzusammensetzung nach Anspruch 1 bis 5 für die Vorbeugung oder terapeutische Behandlung von Autoimmunerkrankungen.

11. Impfstoffzusammensetzung nach Anspruch 1 bis 5 für die Vorbeugung oder terapeutische Behandlung von Allergieerkrankungen.

12. Impfstoffzusammensetzung nach Anspruch 1 bis 5 für die Schwangerschaftsverhütung.

## Revendications

1. Composition de vaccin obtenu à partir de Streptomyces, **caractérisée en ce qu'**elle possède comme principe actif une ou plusieurs souches sauvages du genre Streptomyces ou des souches mutantes ou recombinantes dérivant de ces souches, ainsi qu'un excipient approprié.

2. Composition de vaccin selon la revendication 1, **caractérisée en ce que** les souches de Streptomyces sont des souches vivantes.

3. Composition de vaccin selon les revendications 1 et 2, **caractérisée en ce que** ces souches de Streptomyces sont *Streptomyces lividans,* Streptomyces coelicolor et *Streptomyces Sp.*

4. Composition de vaccin selon la revendication 1, **caractérisée en que** ces souches recombinantes de Streptomyces expriment un ou plusieurs antigènes hétérologues.

5. Composition de vaccin selon la revendication 4, **caractérisée en ce que** ces souches recombinantes de Streptomyces expriment un ou plusieurs antigènes hétérologues de *Mycobacterium Tuberculosis.*

6. Composition de vaccin selon les revendications 1 à 5 pour la prévention ou la thérapeutique de maladies infectieuses.

7. Composition de vaccin selon la revendication 6 pour la prévention ou la thérapeutique anti-mycobactéries.

8. Composition de vaccin selon la revendication 7 pour la prévention ou la thérapeutique contre la tuberculose.

9. Composition de vaccin selon les revendications 1 à 5 pour la prévention ou la thérapeutique contre les maladies tumorales.

10. Composition de vaccin selon les revendications 1 à 5 pour la prévention ou la thérapeutique des maladies auto-immunes.

11. Composition de vaccin selon les revendications 1 à 5 pour la prévention ou la thérapeutique contre les maladies allergiques.

12. Composition de vaccin selon les revendications 1 à 5 pour la prévention de la grossesse.
